# FASCICULE DE BREVET EUROPEEN

(11) **EP 0 804 225 B1**
(45) Date de publication et mention de la délivrance du brevet: **28.11.2001**
(21) Numéro de dépôt: 96901386.1
(22) Date de dépôt: 12.01.1996
(51) Int. Cl.: A61K 38/44

(54) **COMPOSITIONS PHARMACEUTIQUES COMPRENANT UNE SUPEROXYDE DISMUTASE**
PHARMAZEUTISCHE ZUSAMMENSETZUNGEN ENTHALTENT EIN SUPEROXYDDISMUTASE
PHARMACEUTICAL COMPOSITIONS COMPRISING A SUPEROXIDE DISMUTASE

(30) Priorité: 12.01.1995 FR 9500309
(43) Date de publication de la demande: 05.11.1997
(73) Titulaire: Isocell, 92200 Neuilly sur Seine (FR)
(72) Inventeur: POSTAIRE, Eric, F-92170 Vanves (FR); REGNAULT, Corinne, F-92160 Antony (FR); ROCK-ARVEILLER, Monique, F-91120 Palaiseau (FR); STELLA, Valérie, F-77410 Claye-Souilly (FR); BRACK, Michel, F-92270 Bois-Colombes (FR); SAUZIERES, Jacques, F-78470 Saint-Rémy-Les-Chevreuses (FR)
(74) Mandataire: Orès, Bernard
(86) Numéro de dépôt international: FR9600055
(87) Numéro de publication internationale: WO9621462

(56) Documents cités:
- EP-A- 0 292 964
- EP-A- 0 342 620
- DATABASE WPI Week 9223 Derwent Publications Ltd., London, GB; AN 92-189207 & JP,A,04 124 122 (SHISEIDO CO. LTD) , 24 Avril 1992

## Description

La présente invention est relative à de nouvelles compositions pharmaceutiques particulièrement bien adaptées à l'administration orale de la superoxyde dismutase (SOD), en lui assurant une bonne biodisponibilité et une efficacité thérapeutique.

Les superoxyde dismutases font l'objet, depuis leur caractérisation en 1968, par McCord et Fridovich (J. Biol. Chem., 1969, 244, 6049-6055), d'études dans le traitement de nombreuses affections ; en effet, il s'agit d'une enzyme qui favorise l'élimination du radical superoxyde (O₂⁻) par dismutation et constitue donc un système de protection contre les effets délétères de ce radical, susceptible de se former *in vivo,* à partir de l'oxygène atmosphérique. Cette enzyme joue donc un rôle capital dans la prévention des effets toxiques qui pourraient résulter de l'exposition des cellules et de l'organisme à une atmosphère oxygénée où l'oxygène (biradical) perd un électron célibataire (réduction).

Les radicaux libres étant impliqués dans de nombreuses affections, l'utilisation de la SOD en thérapeutique a donc été préconisée dans différents processus inflammatoires (rhumatismes, fibroses, notamment), viraux (infection par le VIH notamment) et dans des conditions toxiques, liées à la présence d'oxygène en quantité importante (système nerveux central, ischémie, désordres gastro-intestinaux non vasculaires, désordres oculaires ou lutte contre les effets indésirables des traitements anti-cancéreux)(Greenwald R.A., *Free Radical Biol. Med.,* 1990, 8, 201-209).

Les formes libres de SOD qui ont été testées, sont la Cu,Zn-SOD (origine végétale ou animale : bovine, rat ou humaine), la Mn-SOD (origine humaine, végétale, algale), la Fe-SOD et les SOD recombinantes.

Les demi-vies plasmatiques des SOD natives sont très variables (de l'ordre de quelques minutes pour la Cu,Zn-SOD ; de l'ordre de plusieurs heures pour la Mn-SOD, par exemple).

Pour augmenter la demi-vie plasmatique de ces SOD, différentes formes modifiées, pour l'administration parentérale ont été proposées ; on peut citer les SOD conjuguées au polyéthylène glycol (SOD-PEG), les SOD conjuguées à l'héparine (SOD-héparine), les SOD conjuguées à l'albumine (SOD-albumine) et les polymères ou copolymères de SOD et les SOD liposomales.

Toutefois ces différentes SOD ont l'inconvénient majeur d'être très peu absorbées lorsqu'elles sont administrées par voie orale.

En conséquence, la Demanderesse s'est donné pour but de mettre au point des formes galéniques aptes à permettre une absorption efficace de SOD par voie orale, une telle voie d'administration étant particulièrement intéressante pour la plupart des affections à traiter précitées.

La présente invention a pour objet des compositions pharmaceutiques, caractérisées en ce qu'elles comprennent essentiellement en combinaison, une superoxyde dismustase et au moins un composé sélectionné dans le groupe constitué par des lipides et des protéines et éventuellement un ou plusieurs véhicules pharmaceutiquement acceptables, lesquelles compositions sont particulièrement bien adaptées à l'administration orale.

Selon un mode de réalisation avantageux desdites compositions, lesdits lipides sont sélectionnés parmi les lipides végétaux, de préférence dans le groupe constitué par les céramides, les phospholipides, les tylacoïdes et les diacylglycérols.

Selon un autre mode de réalisation avantageux desdites compositions, lesdites protéines sont sélectionnées parmi les protéines végétales, de préférence dans le groupe constitué par les prolamines et les films polymériques à base desdites prolamines.

Parmi les véhicules préférés, éventuellement associés, on peut citer les liposomes.

Selon un autre mode de réalisation avantageux de ladite composition, lesdits céramides (ou N-acyl sphingosines) sont d'origine synthétique, animale ou végétale, de préférence d'origine végétale, et sont des dérivés N-acyle acide gras de sphingosine de formule 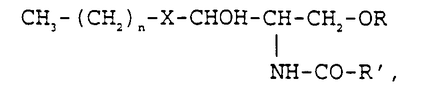 dans laquelle
n est compris entre 5 et 15, de préférence entre 12 et 15,
X représente -CH=CH- ou -CHOH-,
R représente un atome d'hydrogène ou un sucre (glucose, galactose) et
R' représente un groupement alkyle de C₃-C₃₀.

De manière avantageuse, lesdits céramides, d'origine végétale, sont de préférence issus de céréales (farines) et notamment du blé et présentent la formule suivante : 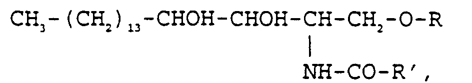 dans laquelle
R représente un atome d'hydrogène ou un glucose,
R' a la même signification que ci-dessus.

De tels céramides végétaux (glycosylés ou non) peuvent notamment être obtenus conformément au procédé décrit dans la Demande Internationale PCT WO 92/00182, au nom des Laboratoires INOCOSM.

Selon un autre mode de réalisation avantageux desdites compositions, les prolamines sont de préférence d'origine végétale et peuvent être obtenues à partir de différentes céréales et notamment à partir du blé, du seigle, de l'orge, de l'avoine, du riz, du millet et du maïs, de préférence à partir du blé (gliadine) et sont de préférence des prolamines natives (c'est-à-dire non dénaturées), issues soit de la farine, soit du gluten frais d'une des céréales précitées.

Selon encore un autre mode de réalisation avantageux desdites compositions, les films polymériques à base de prolamine sont, de préférence, constitués par un polymère hydrophobe comprenant
. au moins une prolamine d'origine végétale,
. au moins un plastifiant choisi dans le groupe constitué par les hydrates de carbone, de préférence les polyols et les esters tels que phtalates, adipates, sébaçates, phosphates, citrates, tartrates et malates, le rapport prolamine:plastifiant étant compris entre 2:1 et 2:0,5 et
. 5 à 30 % d'au moins un solvant choisi parmi les monools, les diols et l'eau et

- en ce qu'ils sont susceptibles d'être obtenus par évaporation d'au moins une fraction de solvant présent dans une composition de départ qui comprend entre 40 et 80 % d'au moins une prolamine en solution dans un solvant hydroalcoolique dont le titre en alcool est compris entre 40 et 80 % et au moins un plastifiant, le rapport plastifiant:solution alcoolique de prolamine étant compris entre 0,10:1 et 0,50:1, de préférence entre 0,20:1 et 0,23:1, jusqu'à l'obtention d'une solution homogène plus ou moins épaisse.

De tels films polymériques, à base de prolamine, peuvent se présenter soit sous forme de gel, soit sous forme de film sec souple ou cassant, c'est-à-dire plus ou moins plastique, selon le degré d'évaporation du solvant.

De manière inattendue, une composition conforme à l'invention est particulièrement bien adaptée à l'administration de SOD par voie orale, car elle augmente de manière significative la biodisponibilité de la SOD, par rapport à celle obtenue, avec les compositions de SOD de l'Art antérieur.

Egalement de manière inattendue, la composition selon l'invention : SOD + film polymérique à base de prolamine (de préférence de la gliadine) :
- protège la SOD à pH acide (milieu gastrique) et
- constitue une forme à libération prolongée.

Outre les dispositions qui précèdent, l'invention comprend encore d'autres dispositions, qui ressortiront de la description qui va suivre, qui se réfère à des exemples de mise en oeuvre du procédé objet de la présente invention ainsi qu'aux dessins annexés, dans lesquels :
- les figures 1 à 3 montrent les concentrations érythrocytaires obtenues après administration de différentes formes de SOD (libre, liposomale, conforme à l'invention : avec céramides), par voie sous-cutanée ;
- les figures 4 à 7 montrent les concentrations érythrocytaires obtenues après administration de différentes formes de SOD, par voie orale ;
- les figures 8 à 15 illustrent les résultats du traitement avec une composition selon l'invention (propriétés anti-inflammatoires obtenues après administration orale), sur le volume de l'oedème de la patte chez le rat (témoin de l'inflammation), en fonction de la quantité de SOD administrée ;
- les figures 16 et 17 illustrent la relation entre la quantité de SOD administrée par voie orale et l'effet anti-inflammatoire (pourcentage d'inhibition de la SOD en fonction du nombre de gavages) ; les oedèmes de la patte sont mesurés 4 h, 5 h et 6 h après les gavages.
- les figures 18 à 20 illustrent l'effet inhibiteur de SOD administrée par voie orale, sur l'activation des polynucléaires et l'augmentation significative de la biodisponibilité avec une composition selon l'invention.

Il doit être bien entendu, toutefois, que ces exemples sont donnés uniquement à titre d'illustration de l'objet de l'invention, dont ils ne constituent en aucune manière une limitation.

### EXEMPLE 1 : Composition conforme à l'invention : SOD + polymère à base de gliadine.

### 1. Méthode d'incorporation de la SOD dans la gliadine :

### * Composition de la formule gliadine + SOD :

| n° | Nom | Quantité |
|---|---|---|
| | Solvant hydroalcoolique | |
| 1 | (50 % v/v d'éthanol) | 18,25 ml |
| 2 | Sorbitol | 1,575 g |
| 3 | Glycérol | 0,675 g |
| 4 | Gliadine | 4,5 g |
| 5 | SOD | 1 ml* |

| | | |
|---|---|---|
| * solutions à : 4 mg/ml, 2 mg/ml, 1 mg/ml. | | |

### * Protocole de fabrication :

Dans un petit bécher placé dans un bain thermostaté à 40°C sous agitation mécanique, introduire le solvant hydroalcoolique. Puis ajouter dans l'ordre les composés 2, 3, 4, 5. Entre chaque addition, laisser agiter jusqu'à dissolution complète des composés.

Le mélange est chauffé et agité afin de diminuer les interactions hydrophobes gliadine-gliadine et donc d'augmenter la solubilité de la gliadine.

Le glycérol est utilisé comme agent hydratant et le sorbitol comme stabilisant et plastifiant. Le mélange obtenu est visqueux, collant et de coloration brunâtre.

### 2. Formulation du mélange obtenu en 1. :

Pour obtenir, par exemple, une composition selon l'invention sous la forme de comprimés, on étale le mélange pâteux obtenu sur un support approprié (support en téflon, en polypropylène, en verre ou en acier inoxydable), on évapore le solvant, soit à 24°C et à un taux d'humidité relative de 60 %, pendant environ une quarantaine d'heures, soit à 60°C et à un taux d'humidité relative de 2 %, pendant une vingtaine d'heures, soit à 37°C : une lampe est placée à 20 cm du film pendant quelques heures (2-10 h).

On obtient un film sec qui est ensuite découpé et pulvérisé de manière à permettre la préparation de comprimés.

### EXEMPLES 2 ET 3 :

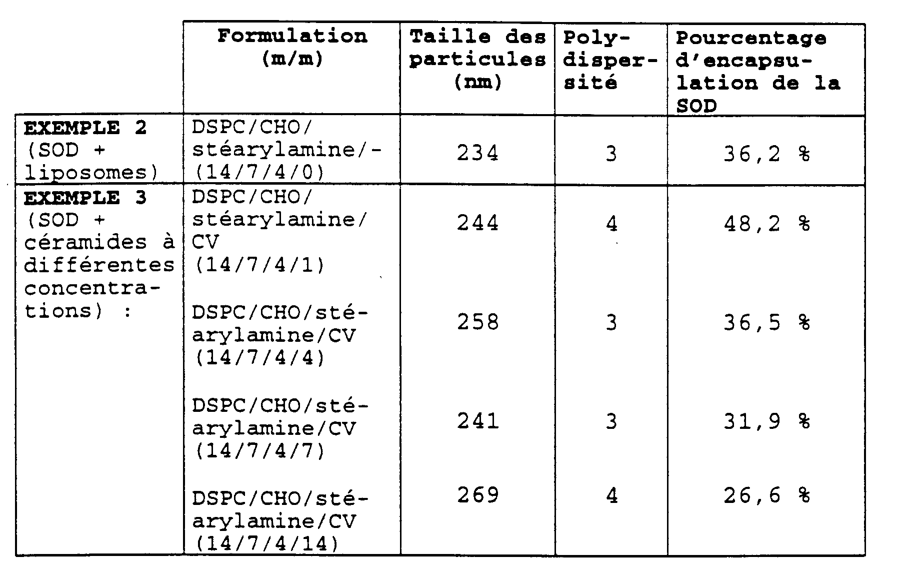

Dans ce tableau , l'abréviation DSPC signifie distéaroyl phosphatidyl choline, l'abréviation CHO signifie cholestérol et l'abréviation CV signifie céramide végétal.

L'exemple 2 correspond à une composition de l'Art antérieur ; l'exemple 3 correspond à une composition conforme à l'invention SOD + céramides, encapsulée pour une part importante dans des liposomes (DSPC/CHO/stéarylamine).

Les liposomes sont obtenus conformément au procédé décrit dans la Demande européenne 0 274 961 ou la Demande européenne 0 349 429 et présentent une bonne homogénéité, comme le montre la colonne polydispersité.

### EXEMPLE 4 : Etude pharmacocinétique comparée après administration par voie sous-cutanée et par voie orale de compositions à base de SOD.

La cinétique de la SOD plasmatique est analysée chez le rat anesthésié après administration par voie orale ou sous-cutanée de SOD libre, de SOD liposomale ou de SOD sous la forme d'une composition selon l'invention.

### - Matériel :

### * Animaux :

Les rats utilisés sont des rats mâles OFA, Sprague-Dawley, dénués de germes pathogènes, pesant de 300 à 400 g, non co-sanguins (élevage IFFA CREDO). Ils sont conservés 3 semaines après leur arrivée à l'animalerie afin d'éviter tout stress lié au changement d'environnement et sont soumis à une diète hydrique 16 à 18 heures avant l'expérience.

### * Matériel utilisé :

· Seringues plastiques graduées à usage unique de 5 ml, pour le gavage (1 par dose), l'injection sous cutanée ainsi que pour l'anesthésie,
· Seringues de 1 ml pour l'anesthésie,
· Canules de gavage 85/14 droite (1 par dose),
· Aiguille 25/0,5 à usage unique, pour l'anesthésie,
· Cristallisoirs pour l'isolement des rats pendant l'endormissement,
· Petit matériel chirurgical pour cathérisation :
   cathéters PE50 (Becton-Dickinson)
   canules trachéales pour aide respiratoire
   robinets 1 voie (Vygon).

### * Solution anesthésiante :

L'anesthésiant utilisé est du thiopental (Nesdonal® ). Il est administré à raison de 50 mg/kg de poids de rat.

La solution est préparée extemporanément.

### * Solution héparinée :

PVP (polyvinylpyrrolidone) hépariné 500 mg/ml + 200 UI d'héparine dans NaCl 0,9 %.

### * Solutions utilisées pour l'étude :

### . Voie orale :

- SOD érythrocytaire bovine (Allerbiodose® ) : 1-2-4 mg/ml, dans du NaCl 0,9 %,
- NaCl 0,9 % (témoin),
- SOD érythrocytaire bovine 1-2-4 mg/ml + céramides végétales 1 % (Inocosm), dans du NaCl 0,9 %, selon l'invention,
- céramides végétales 1 % dans du NaCl 0,9 % (témoin),
- SOD érythrocytaire bovine 1-2-4 mg/ml liposomale,
- liposomes (témoin),
- gliadine extraite du blé (témoin),
- SOD érythrocytaire bovine + gliadine selon l'invention.

### . Voie sous-cutanée :

- SOD érythrocytaire bovine (Allerbiodose® ) 0,5-1-2 mg/ml, dans du NaCl 0,9 %,
- NaCl 0,9 % (témoin),
- SOD érythrocytaire bovine 0,5-1-2 mg/ml + céramides végétales 1 % (Inocosm), dans du NaCl 0,9 % selon l'invention,
- céramides végétales 1 % dans du NaCl 0,9 % (témoin),
- SOD érythrocytaire bovine 0,5-1-2 mg/ml liposomale,
- liposomes (témoin),
- SOD érythrocytaire bovine + gliadine selon l'invention.

### - Méthode :

### * Traitements préalables des animaux :

### . par voie orale : gavage

Le gavage des animaux (n=4) est réalisé à T0 par un volume de 1 ml de solution ou suspension à étudier, dans une solution de NaCl à 0,9 %. Ce gavage est effectué à l'aide d'une canule montée sur une seringue de 1 ml et introduite dans la cavité buccale du rat.

### . par voie sous-cutanée :

L'injection des solutions ou suspensions à étudier est réalisée à T0 par administration d'un volume de 0,3 ml (dans une solution de NaCl à 0,9 %), derrière la tête de l'animal (n=4).

Chaque série d'expérience est réalisée sur 4 animaux par dose. Les groupes témoins étant toujours traités en parallèle pour chaque expérimentation.

### * Cathérisation de la carotide :

Le gavage (ou l'injection sous cutanée) des animaux, une fois réalisé, ces derniers sont anesthésiés par injection lente de thiopental, par voie intra-péritonéale (0,1 ml/100 g).

Lorsque l'animal dort, il est placé sur le dos afin de procéder à la cathérisation de la carotide. Pour cela, la peau est découpée soigneusement au niveau du cou de l'animal à l'aide d'une paire de ciseaux.

Les muscles sont écartés à l'aide d'une pince afin de laisser libre accès à la carotide.

Cette dernière est dégagée, puis clampée côté coeur, et ligaturée côté tête, à l'aide d'un fil fin. Une légère incision de la carotide est alors réalisée à l'aide d'une paire de ciseaux, permettant l'insertion d'un cathéter (sur 1,5 cm environ) monté sur une aiguille équipée d'un robinet une voie. Le cathéter est maintenu en place par une seconde ligature côté coeur. Il est ensuite hépariné à l'aide de la solution héparinée, afin d'éviter toute coagulation du sang lors des prélèvements successifs.

Une compresse de solution de NaCl à 0,9 % est alors posée sur la plaie afin d'éviter un dessèchement excessif de celle-ci.

Pendant toute la durée de l'expérience, les animaux sont placés sous deux lampes afin d'être réchauffés.

### * Prélèvements sanguins :

Les prélèvements sont effectués toutes les heures pendant 6 heures (400 µl), à l'aide du robinet une voie. Le sang est recueilli dans des tubes Eppendorf héparinés (20 µl d'héparine 1 000 UI/ml). Il est ensuite centrifugé 5 min à 4 000 trs/min. Le plasma est alors éliminé et remplacé par du NaCl à 0,9 %. Les tubes sont de nouveau centrifugés 5 min à 4 000 trs/min. Trois lavages successifs des globules rouges sont ainsi réalisés.

### * Dosages érythrocytaires : mesure de l'activité SOD :

Les dosages de l'activité enzymatique de la SOD érythrocytaire (de chacune des formes) sont réalisés sur chaque prélèvement témoins ou traités.

Une dilution adéquate des hématies est réalisée dans de l'eau distillée (+ 0,5 ml d'une solution de triton à 1 %) afin d'obtenir pour le tube essai une inhibition d'environ 50 % par rapport au tube témoin.

Les résultats obtenus en UI de SOD/ml sont rapportés en UI de SOD/mg d'hémoglobine. Le dosage de l'hémoglobine est réalisé par spectrophotométrie à 405 nm.

### - Résultats :

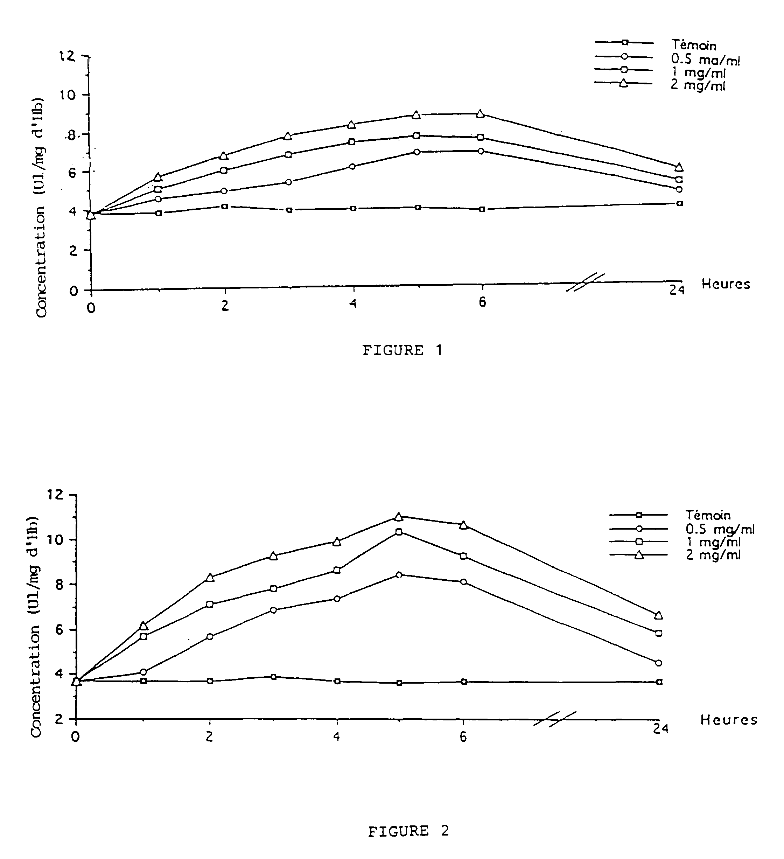

Les résultats des cinétiques érythrocytaires de la SOD administrée par voie sous-cutanée sont présentés aux figures 1-3 :
- la figure 1 correspond aux concentrations érythrocytaires obtenues avec la SOD libre,
- la figure 2 correspond aux concentrations érythrocytaires obtenues avec la SOD liposomale,

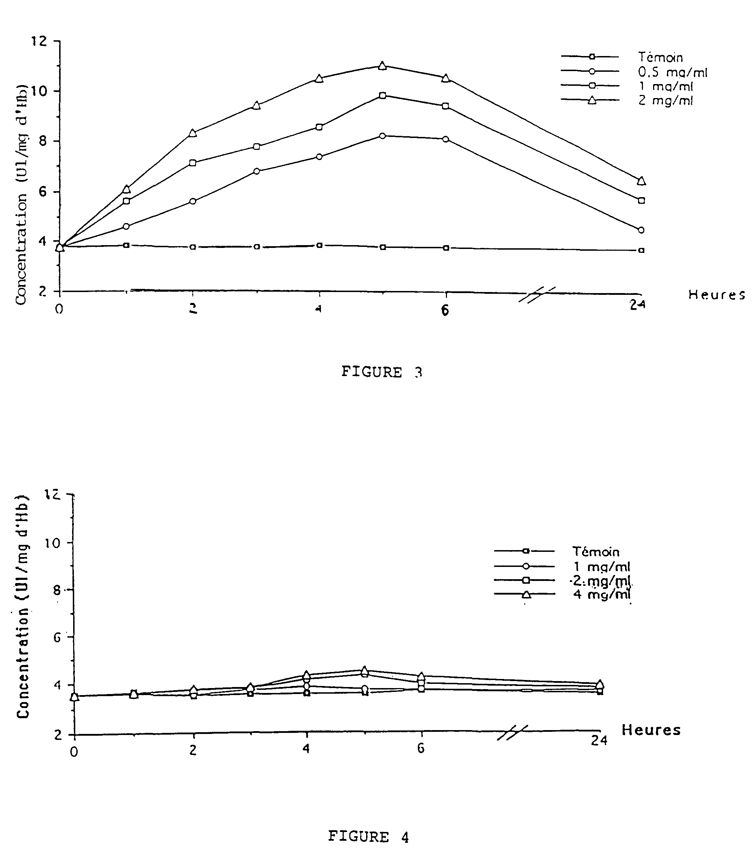

- la figure 3 correspond aux résultats obtenus avec une composition conforme à l'invention : SOD + céramides.

Les résultats des cinétiques érythrocytaires de la SOD administrée par voie orale sont présentés aux figures 4-7 :
- la figure 4 correspond aux concentrations érythrocytaires obtenues avec la SOD libre,

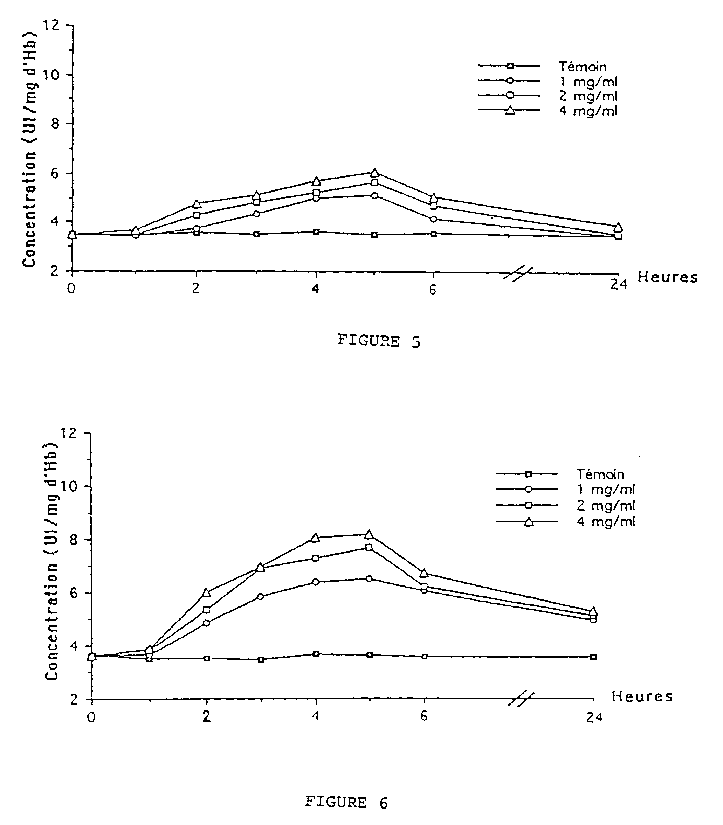

- la figure 5 correspond aux concentrations érythrocytaires obtenues avec la SOD liposomale,
- la figure 6 correspond aux concentrations érythrocytaires obtenues avec une composition conforme à l'invention SOD + céramides et

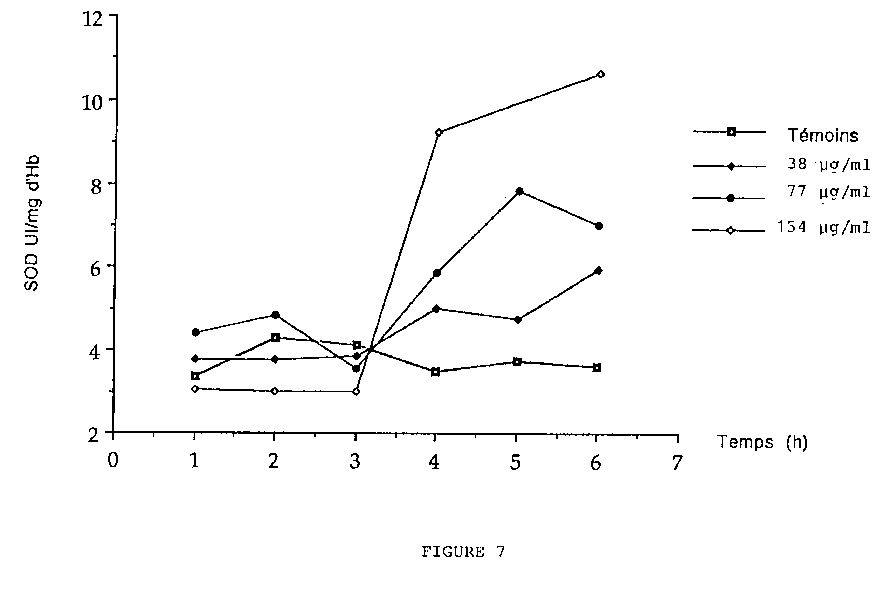

- la figure 7 correspond aux concentrations érythrocytaires obtenues avec une composition conforme à l'invention SOD + gliadine.

Les Tableaux indiquent les données cinétiques obtenues pour les différentes courbes présentées.

**TABLEAU I**

| AUC des courbes de cinétiques érythrocytaires chez le rat, après administration par voie sous-cutanée. | | |
|---|---|---|
| **Courbes cinétiques** | **Concentrations** | **AUC (Ul.h.mg**^{**-1**} **d'Hb)** |
| SOD | 0,5 mg/ml | 42,36 |
| | 1,0 mg/ml | 59,02 |
| | 2,0 mg/ml | 80,88 |
| SOD liposomale (exemple 2) | 0,5 mg/ml | 62,90 |
| | 1,0 mg/ml | 93,69 |
| | 2,0 mg/ml | 118,73 |
| SOD + céramides (exemple 3) | 0,5 mg/ml | 62,28 |
| | 1,0 mg/ml | 91,86 |
| | 2,0 mg/ml | 116,75 |
| ^{·} quantité administrée 0,3 ml. | | |

**TABLEAU II**

| AUC des courbes de cinétiques érythrocytaires chez le rat, après administration par voie orale | | | |
|---|---|---|---|
| **Courbes cinétiques** | **Concentrations (mg/ml)** | **AUC (Ul.h.mg-1 d'Hb)** | **F' (biodisponibilité relative/SC)** |
| SOD | 1,0 | 2,13 | 0,05 |
| | 2,0 | 7,97 | 0,13 |
| | 4,0 | 12,09 | 0,15 |
| SOD liposomale (exemple 2) | 1,0 | 9,90 | 0,16 |
| | 2,0 | 17,24 | 0,18 |
| | 4,0 | 25,87 | 0,22 |
| SOD+céramides (exemple 3) | 1,0 | 45,43 | 0,73 |
| | 2,0 | 52,16 | 0,56 |
| | 4,0 | 61,01 | 0,52 |
| SOD + gliadine (exemple 1) | 1,0 | >20 | ND |
| | 2,0 | >50 | ND |
| | 4,0 | >100 | ND |

| | | | |
|---|---|---|---|
| ND = non déterminable. | | | |

### 5) Conclusion :

### * Voie sous-cutanée :

Les Tₘₐₓ se situent vers 5 heures pour les 4 formes de SOD.

Les Cₘₐₓ sont légèrement plus faibles pour la forme libre, et identiques pour la forme liposome et avec céramides.

Les Cₘₐₓ obtenus avec la SOD + gliadine sont : 6,52 pour une concentration de 25 µg/ml, 7,48 pour une concentration de 50 µg/ml et 65,68 pour une concentration de 100 µg/ml.

### * Voie orale :

Les Tₘₐₓ se situent vers 5 heures pour la SOD libre et les compositions selon les exemples 2 et 3, alors que la formulation à base de gliadine (exemple 1) à un Tₘₐₓ supérieur à 6 h.

Les Cₘₐₓ sont plus faibles pour la forme libre, intermédiaires pour la forme liposomale (exemple 2) et supérieurs pour la forme avec céramides ou gliadine (exemples 1 et 3).

La SOD passe donc à fortes concentrations, par voie orale, et son passage est significativement favorisé lorsqu'une composition selon l'invention est utilisée.

### EXEMPLE 5 : PROPRIETES ANTI-INFLAMMATOIRES DE LA SUPEROXYDE DISMUTASE PAR VOIE ORALE.

### A. Oedème de la patte.

### 1) But de l'étude :

Etudier les cinétiques comparées d'action anti-inflammatoire d'une forme libre de SOD et d'une composition selon l'invention, à différentes concentrations.

### 2) Solutions ou suspensions étudiées (voir exemple 4) :

* SOD libre
* Témoin NaCl
* SOD + céramides (selon l'exemple 3)
* Témoin céramides

### 3) Technique :

### * Traitement des animaux par la SOD :

Gavage des animaux à l'aide d'une canule montée sur une seringue de 0,5 ml insérée dans la cavité buccale du rat, comme décrit à l'exemple 5.

2 gavages sont réalisés par jour (le matin et le soir).

### * Induction de l'oedème :

Injection de 0,1 ml de carragénine λ à 1 % dans le coussinet de la patte.

### * Mesures du volume de la patte :

Les mesures sont réalisées toutes les heures durant 6 heures.

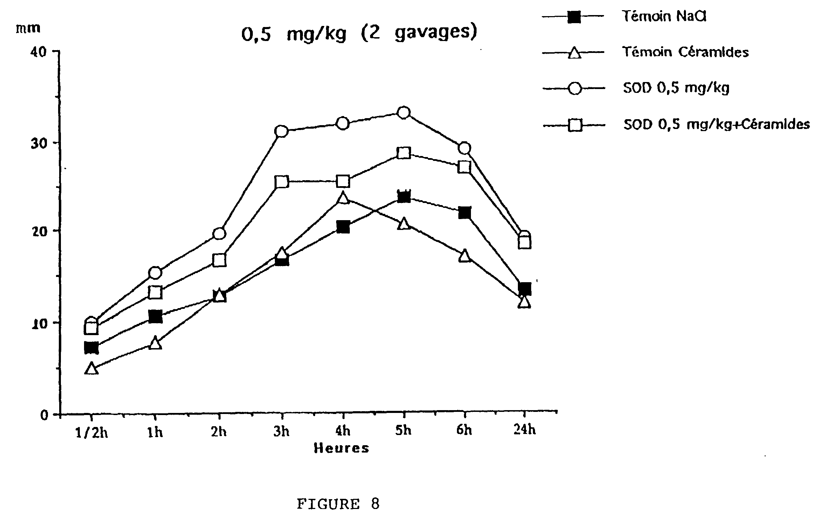

Les figures 8 à 15 illustrent les résultats du traitement avec une composition selon l'invention (propriétés anti-inflammatoires) sur le volume de l'oedème de la patte chez le rat (témoin de l'inflammation), en fonction de la quantité de SOD administrée :
- la figure 8 correspond aux résultats obtenus avec 0,5 mg/kg de SOD (2 gavages),

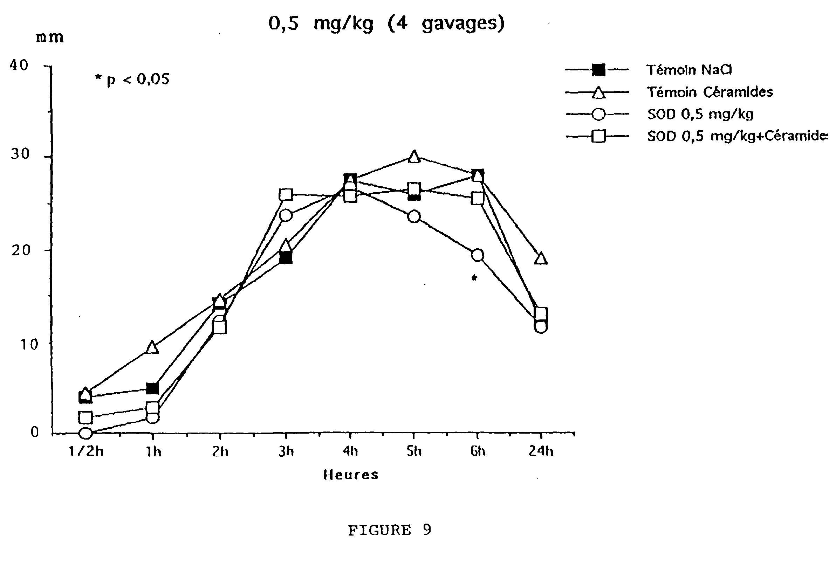

- la figure 9 correspond aux résultats obtenus avec 0,5 mg/kg de SOD (4 gavages),

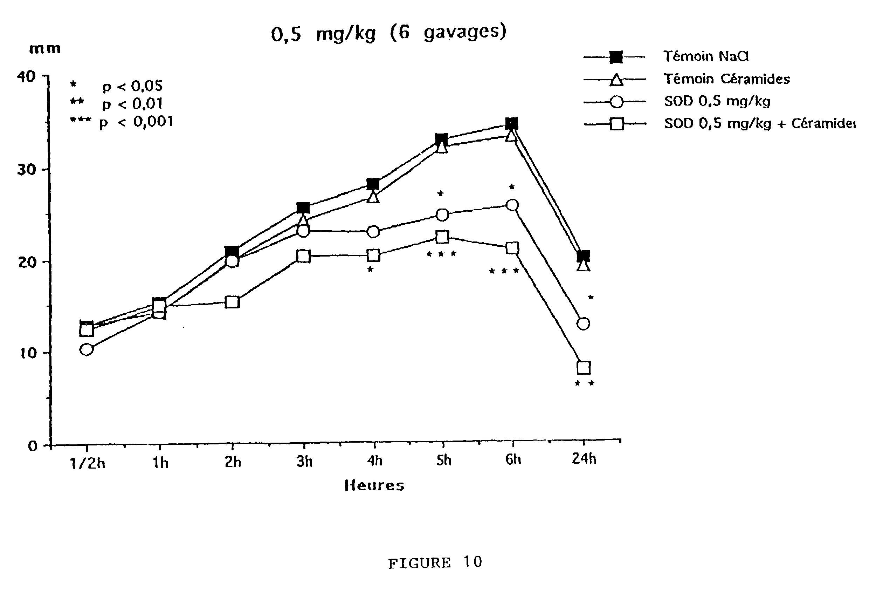

- la figure 10 correspond aux résultats obtenus avec 0,5 mg/kg de SOD (6 gavages),

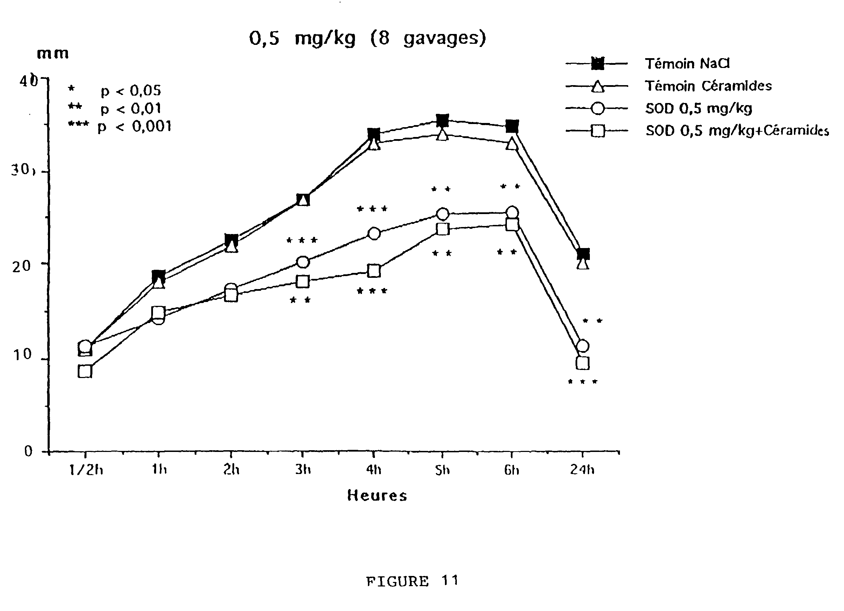

- la figure 11 correspond aux résultats obtenus avec 0,5 mg/kg de SOD (8 gavages),

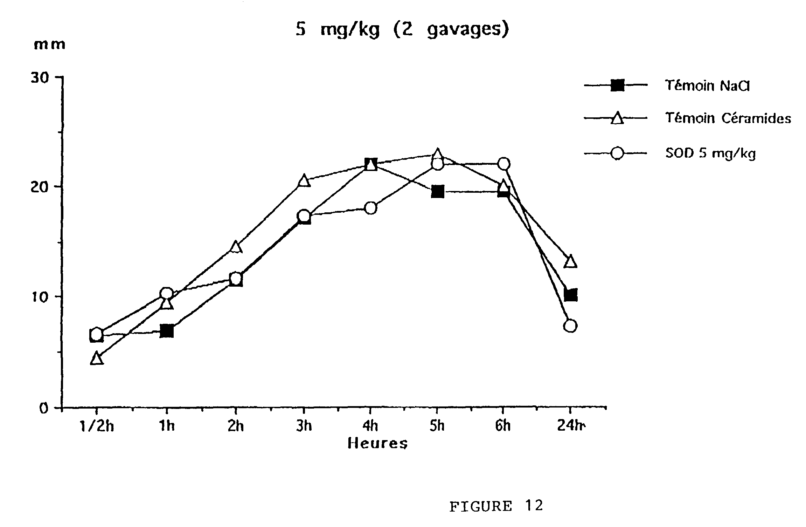

- la figure 12 correspond aux résultats obtenus avec 5 mg/kg de SOD (2 gavages),

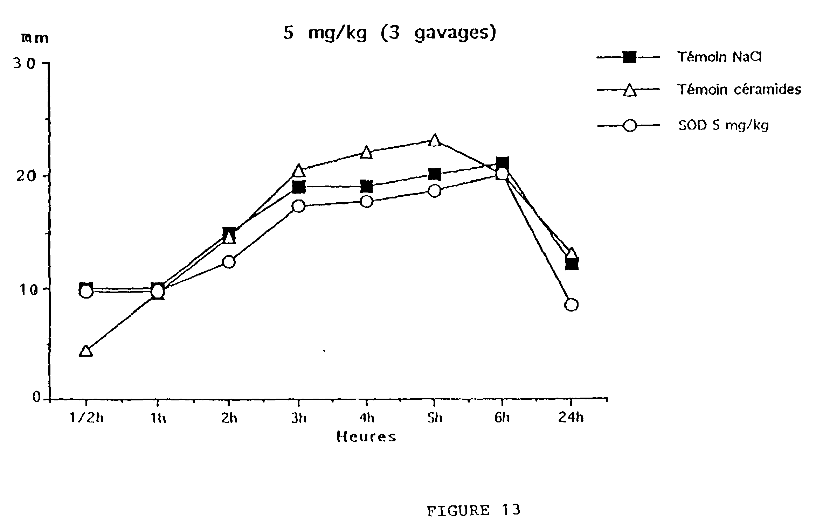

- la figure 13 correspond aux résultats obtenus avec 5 mg/kg de SOD (3 gavages),

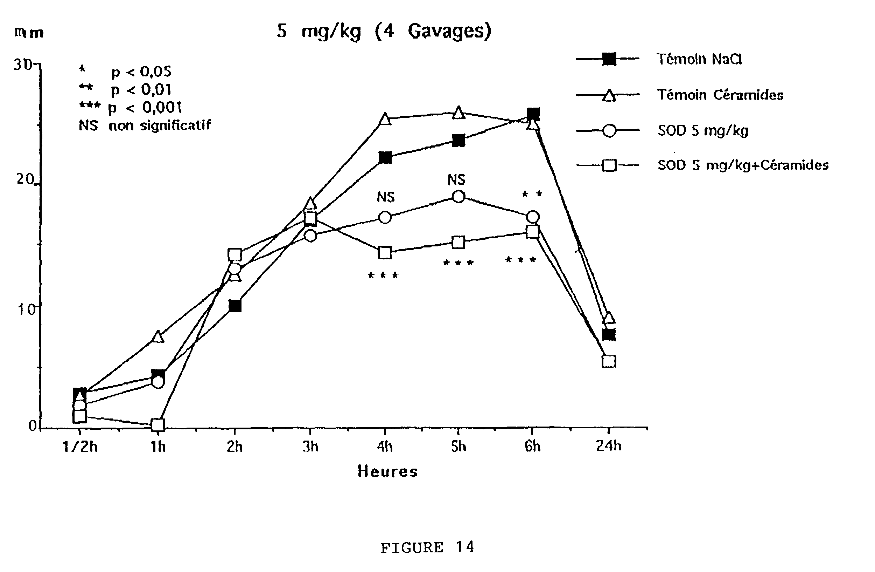

- la figure 14 correspond aux résultats obtenus avec 5 mg/kg de SOD (4 gavages), et

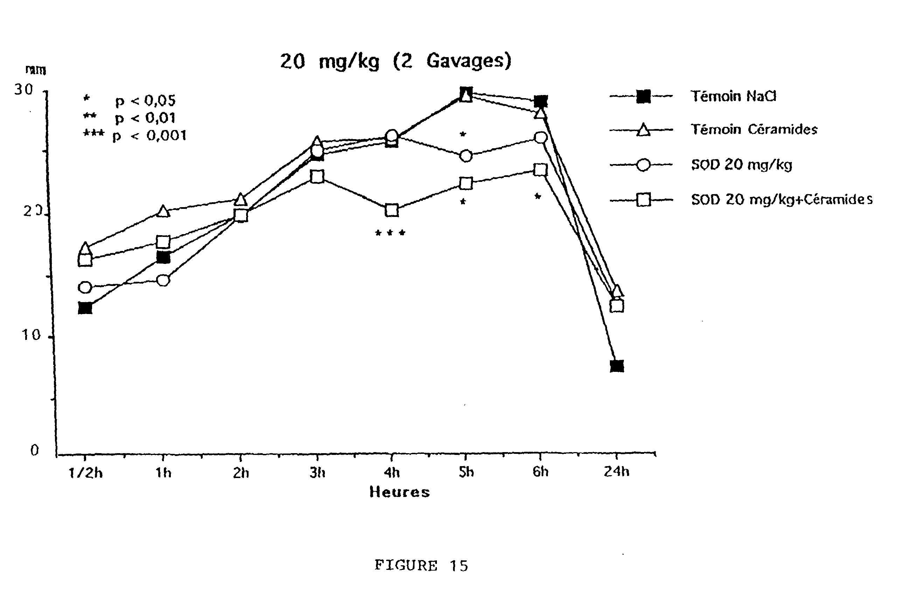

- la figure 15 correspond aux résultats obtenus avec 20 mg/kg de SOD (2 gavages).

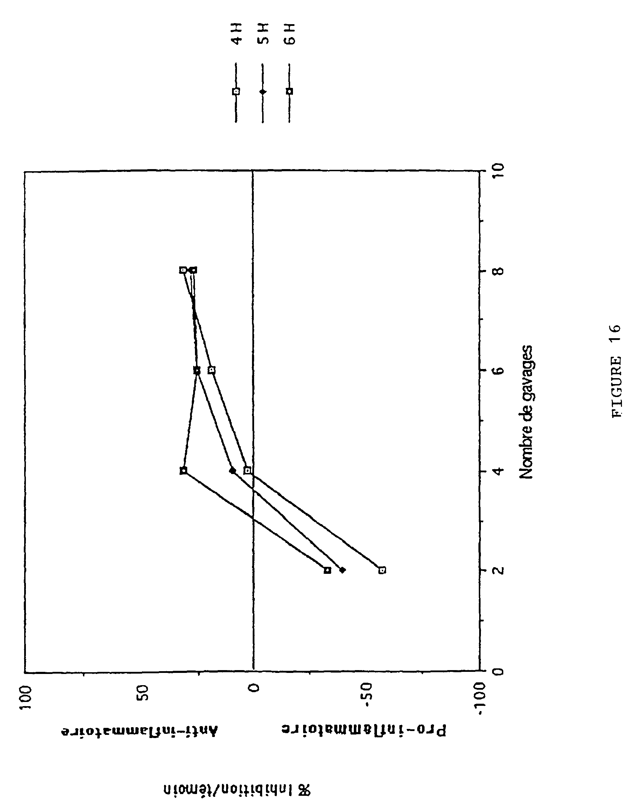

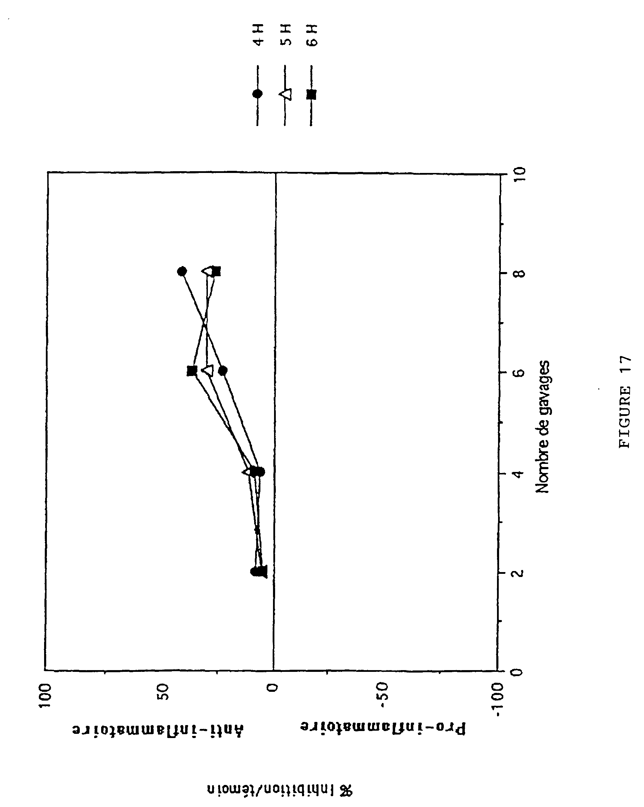

Les figures 16 et 17 illustrent, dans le cadre du même protocole, le pourcentage d'inhibition de la SOD en fonction du nombre de gavages :
- la figure 16 correspond au pourcentage d'inhibition obtenu avec 0,5 mg/kg de SOD,
- la figure 17 correspond au pourcentage d'inhibition obtenu avec 0,5 mg/kg de SOD + céramides.

Les résultats obtenus montrent l'intérêt de l'encapsulation de la SOD pour une activité pharmacologique par voie orale même si une action anti-inflammatoire est démontrée aux mêmes doses à partir de 4 ou 6 gavages pour la SOD libre.

La forme encapsulée de SOD présente toujours un effet anti-inflammatoire, quelque soit le nombre de gavages.

### B. Etude de l'activation des polynucléaires.

### 1) But de l'étude :

comparer l'effet inhibiteur de différentes formes de SOD sur l'activation des polynucléaires.

### 2) Solutions ou suspensions étudiées :

Mêmes solutions ou suspensions que dans l'Exemple 5 A..

### 3) Technique :

### * Prélèvements :

les prélèvements sont ceux recueillis 3 heures après la pleurésie.

### * Dosage :

- Témoin réactif : PBS⁺ (0,65 ml) + cytochrome C 5 mg/ml (0,15 ml).
- Témoin cellule : PBS⁺ (0,65 ml) + cytochrome C 5 mg/ml (0,15 ml) + PN 10 M/ml (0,2 ml).
- Essai : PBS⁺ (0,45 ml) + cytochrome C 5 mg/ml (0,15 ml) + ZO ou PMA (0,2 ml), avec : Témoin NaCl (colonne 1), SOD (colonne 2), Témoin céramides (colonne 3), SOD + céramides (colonne 4), Témoin liposomes (colonne 5), SOD liposomale (colonne 6).
- Incuber 15 minutes au bain marie à 37°C sous agitation,
- arrêter la réaction 10 minutes dans un bain de glace,
- centrifuger 5 minutes à 2 000 trs/min.

### 4) Résultats :

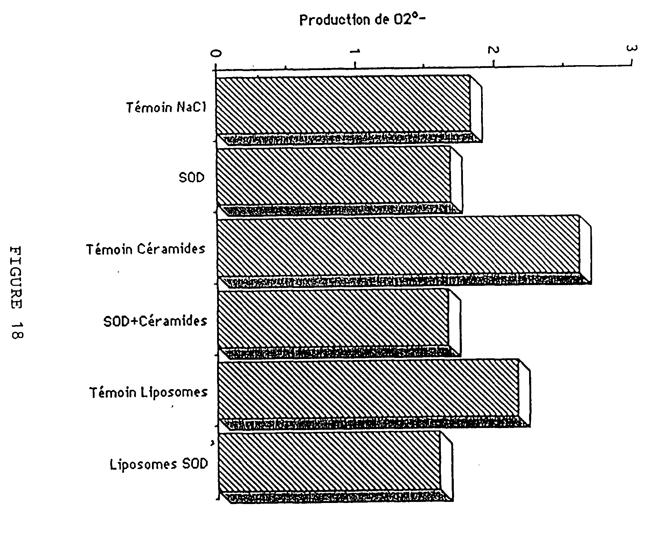

Les figures 18 à 20 illustrent les résultats obtenus :
- la figure 18 montre la production d'anions superoxydes par les polynucléaires après stimulation,

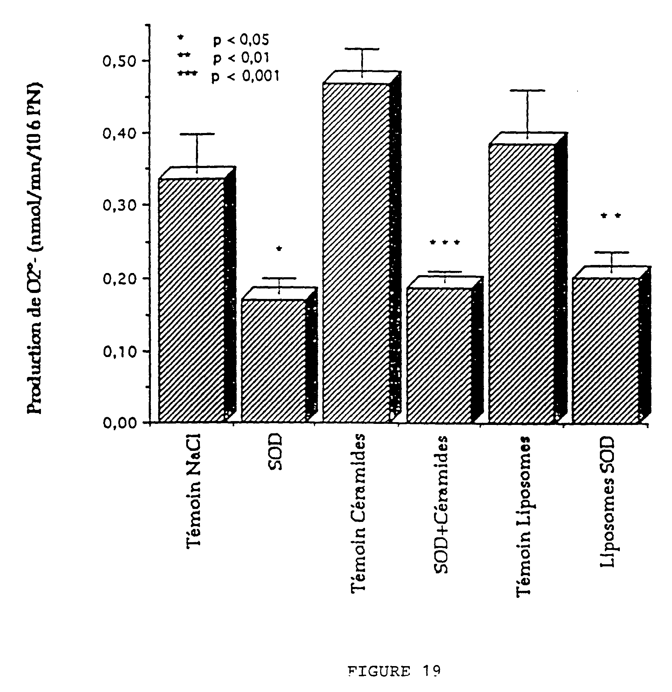

- la figure 19 montre la production d'anions superoxydes par les polynucléaires après stimulation par le zymosan (technique spectrophotométrique),

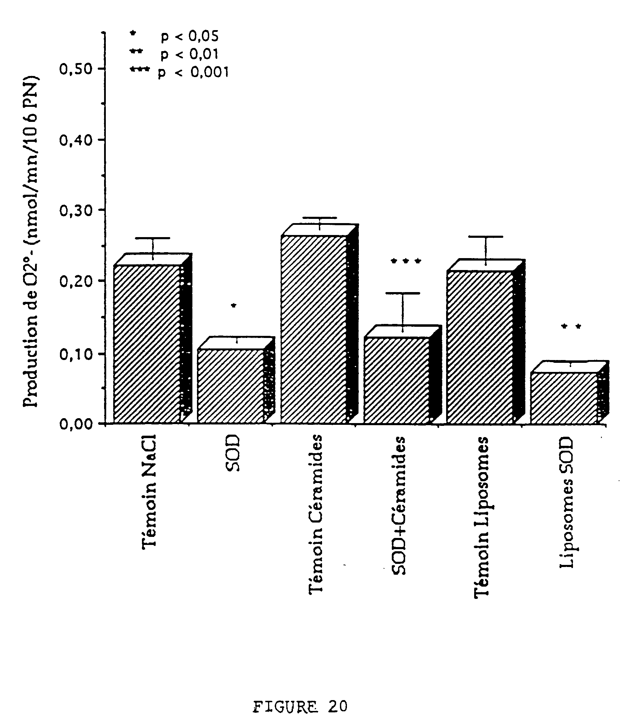

- la figure 20 montre la production d'anions superoxydes par les polynucléaires après stimulation par le PMA (technique spectrophotométrique).

Les résultats montrent de manière significative le rôle antiinflammatoire par voie orale de la SOD libre (P < 0,05), encapsulée par des liposomes (P < 0,07) et encapsulée dans des céramides (P < 0,001).

La figure 17 démontre qu'à la dose de 0,5 mg/kg, l'effet anti-inflammatoire de la SOD libre est observé à partir de 4 gavages ; alors que les résultats de la figure 18 démontrent un effet anti-inflammatoire de la SOD encapsulée dans des céramides dès le second gavage.

## Revendications

1. Compositions pharmaceutiques, **caractérisées en ce qu'**elles comprennent essentiellement en combinaison, une superoxyde dismutase et au moins un composé sélectionné dans le groupe constitué par des lipides sélectionnés dans le groupe constitué par les céramides et des protéines sélectionnées dans le groupe constitué par les prolamines et les films polymériques à base desdites prolamines et éventuellement un ou plusieurs véhicules pharmaceutiquement acceptables.

2. Composition selon la revendication 1, **caractérisée en ce que** lesdits céramides (ou N-acyl sphingosines) sont d'origine végétale et sont des dérivés N-acyle acide gras de sphingosine de formule 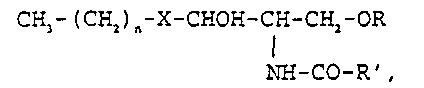 dans laquelle
n est compris entre 5 et 15, de préférence entre 12 et 15,
X représente -CH=CH- ou -CHOH-,
R représente un atome d'hydrogène ou un sucre (glucose, galactose) et
R' représente un groupement alkyle de C₃-C₃₀.

3. Composition selon la revendication 2, **caractérisée en ce que** lesdits céramides, d'origine végétale sont de préférence issus de céréales (farines) et notamment du blé et présentent la formule suivante : 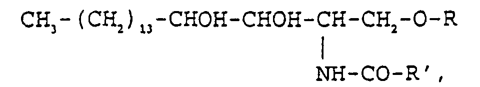 dans laquelle
R représente un atome d'hydrogène ou un glucose,
R' a la même signification que ci-dessus.

4. Composition selon la revendication 1, **caractérisée en ce que** lesdites prolamines sont d'origine végétale et sont issues de différentes céréales choisies dans le groupe constitué par du blé, du seigle, de l'orge, de l'avoine, du riz, du millet et du maïs.

5. Composition selon la revendication 1, **caractérisée en ce que** les films polymériques à base de prolamine sont, de préférence, constitués par un polymère hydrophobe comprenant
. au moins une prolamine d'origine végétale,
. au moins un plastifiant choisi dans le groupe constitué par les hydrates de carbone, de préférence les polyols et les esters tels que phtalates, adipates, sébaçates, phosphates, citrates, tartrates et malates, le rapport prolamine:plastifiant étant compris entre 2:1 et 2:0,5 et
. 5 à 30 % d'au moins un solvant choisi parmi les monools, les diols et l'eau et
- **en ce qu'**ils sont susceptibles d'être obtenus par évaporation d'au moins une fraction de solvant présent dans une composition de départ qui comprend entre 40 et 80 % d'au moins une prolamine en solution dans un solvant hydroalcoolique dont le titre en alcool est compris entre 40 et 80 % et au moins un plastifiant, le rapport plastifiant:solution alcoolique de prolamine étant compris entre 0,10:1 et 0,50:1, de préférence entre 0,20:1 et 0,23:1, jusqu'à l'obtention d'une solution homogène plus ou moins épaisse.

6. Composition selon l'une quelconque des revendications 1 à 5, destinée à une administration par voie orale.

7. Composition selon l'une quelconque des revendications 1 à 6, **caractérisée en ce qu'**elle comprend en tant que véhicules associés, des liposomes.

8. Utilisation d'une composition selon l'une quelconque des revendications 1 à 7, pour la préparation d'un médicament destiné au moins au traitement de l'inflammation.

## Patentansprüche

1. Pharmazeutische Zusammensetzungen, **dadurch gekennzeichnet, daß** sie im wesentlichen in Kombination eine Superoxiddismutase sowie mindestens eine Verbindung, die aus der Gruppe bestehend aus Lipiden ausgewählt ist, die aus der Gruppe bestehend aus Ceramiden und Proteinen ausgewählt sind, die aus der Gruppe bestehend aus Prolaminen und Polymerfilmen auf Basis der Prolamine ausgewählt sind, und gegebenenfalls ein oder mehrere pharmazeutisch akzeptable Vehikel aufweisen.

2. Zusammensetzung nach Anspruch 1, **dadurch gekennzeichnet, daß** die Ceramide (bzw. N-Acyl-Sphingosine) pflanzlichen Ursprungs sind und N-Fettsäureacylderivate von Sphingosin mit der Formel 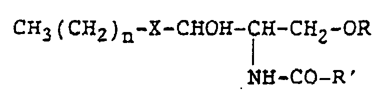 sind, in der
n zwischen 5 und 15, vorzugsweise zwischen 12 und 15 beträgt,
X für -CH=CH- oder -CHOH- steht,
R für ein Wasserstoffatom oder einen Zucker (Glucose, Galactose) steht, und
R' für eine C₃-C₃₀-Alkylgruppierung steht.

3. Zusammensetzung nach Anspruch 2, **dadurch gekennzeichnet, daß** die Ceramide pflanzlichen Ursprungs vorzugsweise von Getreiden (Mehlen) und insbesondere von Weizen stammen und die folgende Formel aufweisen: 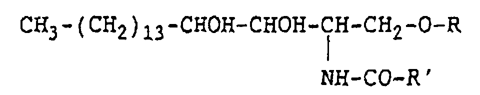 in der
R für ein Wasserstoffatom oder eine Glucose steht,
R' die gleiche Bedeutung wie obenstehend angegeben hat.

4. Zusammensetzung nach Anspruch 1, **dadurch gekennzeichnet, daß** die Prolamine pflanzlichen Ursprungs sind und von verschiedenen Getreiden stammen, die aus der Gruppe bestehend aus Weizen, Roggen, Gerste, Hafer, Reis, Hirse und Mais ausgewählt sind.

5. Zusammensetzung nach Anspruch 1, **dadurch gekennzeichnet, daß** die Polymerfilme auf Prolaminbasis vorzugsweise aus einem hydrophoben Polymer bestehen, welches aufweist:
- mindestens ein Prolamin pflanzlichen Ursprungs,
- mindestens einen Weichmacher, der aus der Gruppe bestehend aus den Kohlehydraten, vorzugsweise Polyolen und Estern wie Phthalaten, Adipaten, Sebacaten, Phosphaten, Citraten, Tartraten und Malaten ausgewählt ist, wobei das Verhältnis Prolamin : Weichmacher zwischen 2 : 1 and 2 : 0,5 liegt, und
- 5 bis 30% mindestens eines unter den einwertigen Alkoholen, Diolen und Wasser ausgewählten Lösungsmittels, sowie
- dadurch, daß sie erhältlich sind, indem mindestens eine Lösungsmittelfraktion, welche in einer Ausgangszusammensetzung vorhanden ist, die zwischen 40 und 80% mindestens eines Prolamins, das in Lösung in einem Wasser-Alkohollösungsmittel vorliegt, dessen Alkoholgehalt zwischen 40 und 80% beträgt, sowie mindestens einen Weichmacher aufweist, wobei das Verhältnis Weichmacher : alkoholische Prolaminlösung zwischen 0,10 : 1 und 0,50 : 1, vorzugsweise zwischen 0,20 : 1 und 0,23 : 1 beträgt, bis zum Erhalt einer mehr oder weniger dicken homogenen Lösung eingeengt wird.

6. Zusammensetzung nach einem der Ansprüche 1 bis 5, die für eine Verabreichung auf oralem Wege bestimmt ist.

7. Zusammensetzung nach einem der Ansprüche 1 bis 6, **dadurch gekennzeichnet, daß** sie als zugeordnete Vehikel Liposome aufweist.

8. Verwendung einer Zusammensetzung nach einem der Ansprüche 1 bis 7 zur Herstellung eines zumindest für die Behandlung von Entzündungen bestimmten Medikaments.

## Claims

1. Pharmaceutical compositions, **characterized in that** they essentially comprise a combination of a superoxyde dismutase and at least one compound selected from the group consisting of lipids, selected from the group consisting of ceramides, and proteins, selected from the group consisting of prolamines and polymer films based on said prolamines, and optionally one or more pharmaceutically acceptable vehicles.

2. Composition according to claim 1, **characterized in that** said ceramides (or N-acylsphingosines) are of vegetable origin and are N-acyl fatty acid derivatives of sphingosine of formula 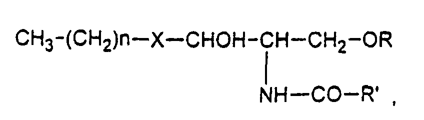 in which
n is between 5 and 15, preferably between 12 and 15,
X is -CH=CH- or -CHOH-
R is a hydrogen atom or a sugar (glucose, galactose) and
R' is a C₃-C₃₀ alkyl group.

3. Composition according to claim 2, **characterized in that** said ceramides of vegetable origin are preferably derived from cereals (flours), especially wheat, and have the following formula: 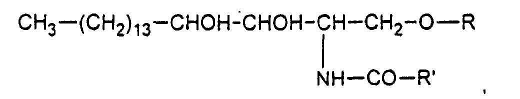 in which
R is a hydrogen atom or a glucose and
R' is as defined above.

4. Composition according to claim 1, **characterized in that** said prolamines are of vegetable origin and are derived from different cereals selected from the group consisting of wheat, rye, barley, oats, rice, millet and maize.

5. Composition according to claim 1, **characterized in that**
- the polymer films based on prolamine preferably consist of a hydrophobic polymer comprising
. at least one prolamine of vegetable origin,
. at least one plasticizer selected from the group consisting of carbohydrates, preferably polyols and esters such as phthalates, adipates, sebacates, phosphates, citrates, tartrates and malates, the ratio of prolamine to plasticizer being between 2:1 and 2:0.5, and
. 5 to 30% of at least one solvent selected from monools, diols and water,
- and **in that** they can be obtained by evaporation of at least part of the solvent present in a starting composition comprising between 40 and 80% of at least one prolamine dissolved in an aqueous-alcoholic solvent with an alcohol titre of between 40 and 80%, and at least one plasticizer, the ratio of plasticizer to alcoholic prolamine solution being between 0.10:1 and 0.50:1, preferably between 0.20:1 and 0.23:1, until a homogeneous solution of greater or lesser thickness is obtained.

6. Composition according to any one of claims 1 to 5, intended for oral administration.

7. Composition according to any one of claims 1 to 6, **characterized in that** it comprises liposomes as associated vehicles.

8. Use of a composition according to any one of claims 1 to 7 for the preparation of a drug intended at least for the treatment of inflammation.
